(19) Europäisches Patentamt
European Patent Office
Office européen des brevets

(11) Numéro de publication : **0 434 546 B1**

# (12) FASCICULE DE BREVET EUROPEEN

(45) Date de publication du fascicule du brevet : **19.05.93 Bulletin 93/20**

(51) Int. Cl.$^5$ : **C07D 301/12,** C07D 303/16

(21) Numéro de dépôt : **90403648.0**

(22) Date de dépôt : **18.12.90**

## (54) Procédé d'époxydation sélective de composés (méth)acryliques insaturés et nouveaux (méth)acrylates bifonctionnels obtenus.

(30) Priorité : **22.12.89 FR 8917134**

(43) Date de publication de la demande : **26.06.91 Bulletin 91/26**

(45) Mention de la délivrance du brevet : **19.05.93 Bulletin 93/20**

(84) Etats contractants désignés : **AT BE CH DE DK ES FR GB GR IT LI LU NL SE**

(56) Documents cités :
**EP-A- 0 190 609**
**DE-A- 3 602 254**
**GB-A- 2 055 821**
**CHEMICAL ABSTRACTS, vol. 108, no. 16, 18 avril 1988, page 8, résumé no. 132450e, Columbus, Ohio, US; & JP-A-62 81 378 (SANYO KOKUSAKU PULP CO., LTD) 14-04-1987**
**J.Org.Chem. 48 (1983) p. 3831-3833**

(73) Titulaire : **ELF ATOCHEM S.A.**
**4 & 8, Cours Michelet La Défense 10**
**F-92800 Puteaux (FR)**

(72) Inventeur : **Caubere, Paul**
**11, rue de Serre**
**F-54000 Nancy (FR)**
Inventeur : **Fort, Yves**
**7, Square de Liège**
**F-54500 Vandoeuvre Les Nancy (FR)**
Inventeur : **Otar, Agnès**
**22, route de Giraumont**
**F-54800 Jarny (FR)**

(74) Mandataire : **Rieux, Michel et al**
**ATOCHEM Département Propriété Industrielle**
**4, Cours Michelet - La Défense 10 - Cedex 42**
**F-92091 Paris-La Défense (FR)**

EP 0 434 546 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à un procédé d'époxydation sélective de composés (méth)acryliques insaturés.

La réaction d'époxydation de composés organiques insaturés au moyen de peroxyde d'hydrogène et d'un sel d'un acide de métal lourd est bien connue depuis longtemps. Ainsi, les brevets US-A-2 833 787 et 2 833 788 ont décrit l'époxydation de composés éthyléniques non conjugués, par exemple d'alcools monoéthyléniques, au moyen de peroxyde d'hydrogène et de pertungstate de sodium à un pH compris entre 3 et 7. De même, l'article publié dans J. Org. Chem., vol.24, pages 54-55 (janvier 1959) décrit l'époxydation d'acides $\alpha,\beta$-insaturés au moyen de peroxyde d'hydrogène et de tungstate de sodium à un pH compris entre 4 et 5,5.

Il est également connu par Angew. Chem. Int. Ed. Engl. 21 (1982) 734-750 l'époxydation d'oléfines au moyen de peroxyde d'hydrogène et de composés de molybdène. De même, J. Org. Chem. (1983), vol. 48, 3831-3833 décrit l'époxydation d'oléfines à 70°C dans des conditions de catalyse par transfert de phase au moyen de peroxyde d'hydrogène dilué (à moins de 10%) et de tungstate alcalin soluble dans l'eau, à pH 1,6 et en présence d'un agent de transfert de phase tel qu'un halogénure d'ammonium ou de phosphonium quaternaire, le rapport molaire du peroxyde d'hydrogène à l'oléfine étant égal à 0,6. L'article publié par J. Org. Chem. (1985) vol. 50, 2688-2690 décrit une réaction similaire effectuée à 50°C en présence de complexes de molybdène et de ligands monodentates, le rapport molaire de peroxyde d'hydrogène à l'oléfine étant égal à 0,2.

Enfin, il est connu d'époxyder les oléfines à 60°C dans le chloroforme, en présence d'un catalyseur formé à partir d'acide molybdophosphorique ou tungstophosphorique et de chlorure de cétylpyridinium, au moyen de peroxyde d'hydrogène concentré (35%) et le rapport molaire du peroxyde d'hydrogène à l'oléfine étant égal à 1,5 ou à 1 : on pourra lire à ce sujet J. Org. Chem. (1987), vol. 52, 1868-1870 et J. Org. Chem. (1988), vol. 53, 3587-3593.

Par ailleurs, le brevet US-A-3 459 775 décrit l'époxydation de (méth)acrylate de vinylnorbornyle à la température de 50°C, pendant une durée de 7 à 9 heures, au moyen d'acide peracétique. Le 2-époxyéthylbicyclo[2.2.1] hept-5(6)-yl (méth)acrylate est obtenu dans ces conditions avec un rendement ne dépassant pas 42%. De même, la demande de brevet JP-A-62/81 378 décrit l'époxydation de l'acrylate de dicyclopentenyloxyéthyle à la température de 60°C, pendant 2 heures, au moyen de peroxyde d'hydrogène à une concentration de 35%. L'acrylate époxydé est obtenu dans ces conditions avec un rendement ne dépassant pas 48%.

La demande de brevet britannique GB-A-2 055 821 décrit un procédé d'époxydation catalytique d'oléfines par réaction avec $H_2O_2$ selon la technique à double phase aqueuse-organique liquide avec des sels d'onium, en présence d'un système catalytique constitué par au moins un élément choisi parmi le tungstène, le molybdène et le vanadium et leurs dérivés, et au moins un dérivé du phosphore ou de l'arsenic.

La demande de brevet européen EP-A-0 190 609 décrit un procédé de monoépoxydation de composés contenant deux doubles liaisons oléfiniques par $H_2O_2$ à une concentration de 3 à 70%, procédé suivant lequel on place les deux réactifs en présence d'un catalyseur constitué par une zéolite synthétique.

Le problème que la présente invention s'est proposé de résoudre consiste, compte tenu des enseignements de l'art antérieur rappelé ci-dessus relatifs à l'époxydation de composés organiques à insaturation éthylénique tels que des oléfines, des alcools, des acides ou certains (méth)acrylates, à mettre au point les conditions de l'époxydation sélective d'insaturations non acryliques de composés (méth)acryliques insaturés au moyen de peroxyde d'hydrogène.

Dans ce contexte, un premier objet de la présente invention consiste en un procédé d'époxydation d'un composé (méth)acrylique insaturé de formule:

(I)

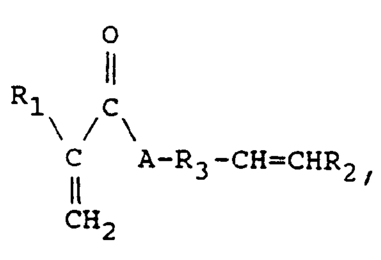

dans laquelle :

- $R_1$ est choisi parmi l'atome d'hydrogène et les radicaux alkyle ayant de 1 à 5 atomes de carbone,
- $R_2$ est choisi parmi l'atome d'hydrogène, les radicaux alkyle, (notamment ceux ayant de 1 à 12 atomes

de carbone) et les radicaux aryle (notamment ceux ayant de 6 à 14 atomes de carbone),

- $R_3$ est un radical alkylène linéaire, ramifié ou cyclique, ayant de 1 à 12 atomes de carbone ou un radical oxyalkylène de formule $-(CH_2)_m-O-R_4-$, avec $1 \leqq m \leqq 11$ et $R_4$ désignant un radical alkylène linéaire, ramifié ou cyclique, l'un des atomes de carbone de $R_2$ lorsque celui-ci est un radical alkyle pouvant être relié à un des atomes de carbone de $R_3$.
- A est choisi parmi les atomes d'oxygène et de soufre et les radicaux NH et $NR_5$, $R_5$ étant un groupe alkyle ayant de 1 à 12 atomes de carbone

par action de peroxyde d'hydrogène sur ledit composé (méth) acrylique insaturé, à une température comprise entre 10°C et 50°C environ, en présence d'au moins un catalyseur choisi parmi les molybdates et les tungstates alcalins et en présence d'au moins un agent de transfert de phase.

Un très grand nombre de composés (méth)acryliques insaturés peuvent être époxydés conformément au procédé selon l'invention. Ils sont représentés par la formule générale ci-dessus, dans laquelle plus précisement :

$R_2$ peut être par exemple l'atome d'hydrogène, un radical méthyle, éthyle n-propyle, isopropyle, n-butyle, isobutyle, tertio-butyle, n-pentyle, n-hexyle, cyclohexyle, n-octyle, n-décyle, n-dodécyle, phényle, totuyle, xylyle ou naphtyle,

- $R_3$ peut être par exemple un radical méthylène ou polyméthylène

$$-(CH_2)_n-$$

avec $2 \leqq n \leqq 12$, un radical alkylène comportant un ou plusieurs cycles tels que les radicaux dicyclopentényle ou norbornyle, un radical oxyalkylène, $(CH_2)_m-O-R_4$ avec $1 \leqq m \leqq 11$ et $R_4$ désignant un radical alkylène pouvant comporter un ou plusieurs cycles comme indiqué précédemment, et

- l'un des atomes de carbone de $R_2$, lorsque celui-ci est un radical alkyle, peut être lié à l'un des atomes de carbone de $R_3$ pour former un cycle.

De préference, le composé (méth)acrylique insaturé est stabilisé, avant réaction, par addition d'un composé tel que l'hydroquinone, le monoéther méthylique de l'hydroquinone, la phénothiazine, la N,N-diéthylhydroxylamine, le nitrobenzène, le di-tertiobutylcatéchol, le p-anilinophénol, le phosphite de di-(2-éthylhexyl)-octylphényle et leurs mélanges en toutes proportions.

La réaction qui se trouve à la base du procédé selon l'invention doit être effectuée à une température modérée en évitant les températures supérieures à 50°C environ, qui sont souvent la cause de la polymérisation du composé (méth)acrylique insaturé ou de son époxyde, et les températures inférieures à 10°C environ, pour des raisons cinétiques. La réaction selon l'invention est effectuée de préference en présence d'un solvant, ou d'un mélange de solvants organique qui peut être choisi notamment parmi les solvants chlorés tels que le dichloroéthane le chloroforme, le tétrachlorure de carbone, le trichlorométhane et le dichlorométhane, et en quantité telle que le rapport en volume composé à époxyder/solvant soit supérieur à 1,5 environ.

La réaction selon l'invention est effectuée en présence d'un ou plusieurs agents de transfert de phase pouvant être choisis notamment dans la famille des sels, par exemple, des halogénures d'ammonium quaternaire tels que le chlorure de tricaprylylméthylammonium, les chlorure, bromure et iodure de tétrabutylammonium, ou encore dans la famille des sels, par exemple, des halogénures de phosphonium quaternaire tels que les chlorure et bromure de tétrabutylphosphonium et le bromure de tributylhexadécylphosphonium, ou encore pouvant être l'hydrogénosulfate de tétrabutylammonium. L'agent de transfert de phase est utilisé de préférence en une proportion molaire, par rapport au composé à époxyder, au moins égale à 0,5% et plus particulièrement comprise entre 1% et 3%.

Dans le procédé selon l'invention, le rapport molaire entre le peroxyde d'hydrogène et le composé (méth)acrylique insaturé a une grande importance: il est d'au moins une mole, de préférence 1,5 à 3 moles, de peroxyde d'hydrogène, pour une mole de composé (méth)acrylique insaturé. De même, la concentration du peroxyde d'hydrogène, utilisé en solution aqueuse, n'est pas sans influence sur le rendement et sur la sélectivité de la réaction d'époxydation: elle est de préférence comprise entre 5% et 50% environ, et plus particulièrement comprise entre 10% et 35%. Une autre condition réactionnelle importante est le pH du milieu : il sera de préférence choisi entre 1 et 3,5, et plus particulièrement entre 1,5 et 2,5, et sera ajusté au moyen de la quantité nécessaire d'un acide tel que l'acide sulfurique et/ou l'acide phosphorique.

Enfin, la réaction d'époxydation est effectuée en présence d'au moins un catalyseur choisi parmi les molybdates et tungstates alcalins de formule $M_2 M'O_4$ dans laquelle M est un métal alcalin choisi parmi le sodium, le lithium, le potassium, le césium et le rubidium et M est un métal de transition choisi parmi le molybdène et le tungstène. Ce catalyseur est utilisé de préférence en une proportion molaire, par rapport au composé à époxyder, au moins égale à 0,1% et plus particulièrement comprise entre 0,5% et 5%. Le catalyseur peut également être modifié par l'addition d'un acide, de préférence l'acide phosphorique

La réaction selon l'invention conduit, avec un taux de conversion le plus souvent supérieur à 70%, de manière sélective à l'époxyde de formule

(II) 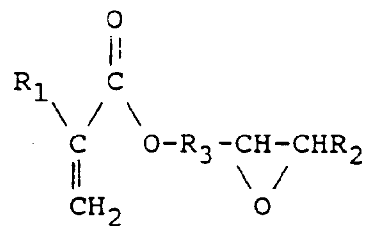

dans laquelle $R_1$, $R_2$ et $R_3$ ont les significations précitées. Celui-ci est formé préférentiellement à l'époxyde de formule:

(III) 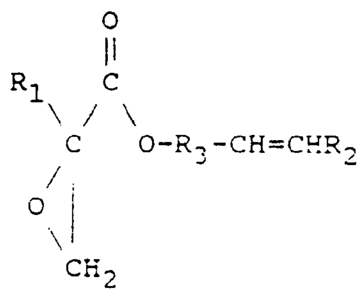

et aux diols correspondants qui peuvent éventuellement en constituer les sous-produits. Ce résultat de l'invention est particulièrement surprenant dans la mesure où jusqu'ici, conformément aux documents antérieurs déjà cités, l'époxydation de (méth)acrylates insaturés procédait avec de faibles rendements malgré des températures réactionnelles plus élevées que celles préconisées par la présente invention.

Les (méth)acrylates obtenus selon l'invention, en raison de leur faible odeur et de leur basse viscosité, pourront trouver des applications en tant qu'agents de modification dans les domaines des encres, des adhésifs, des peintures, des revêtements et des résines.

Les exemples suivants sont donnés à titre illustratif et non limitatif de la présente invention.

## EXEMPLE 1

Une solution constituée de 1,5 millimole de tungstate de sodium $Na_2WO_4$, $2H_2O$, de 2,55 millimoles d'acide phosphorique 34% et de 120 millimoles de peroxyde d'hydrogène 20% aqueux, dont le pH est ajusté à la valeur indiquée au tableau I ci-après par une solution d'acide sulfurique 30%, est additionnée goutte à goutte, en 15 minutes, à une solution constituée de 0,6 millimole de chlorure de tricaprylylméthylammonium (commercialisé sous la dénomination ALIQUAT 336 par la société ALDRICH), et de 60 millimoles de méthacrylate d'allyle (commercialisé par la demanderesse) dans 15 ml de chlorure de méthylène. Le mélange est placé sous vive agitation à la température T (exprimée en degrés Celsius) indiquée au tableau I ci-après. Quelques minutes après l'addition, une coloration jaune apparaît puis s'atténue. La réaction est suivie en chromatographie en phase gazeuse par analyse de prélèvements réguliers. A l'issue du temps t (exprimé en heures) indiqué dans le tableau I ci-après, une solution acide de sulfate de fer est ajoutée au mélange réactionnel afin de détruire les peroxydes présents dans le milieu. A ce stade sont calculés le taux de conversion de la réaction C (exprimé en %) ainsi que les sélectivités S en époxyde de formule (II) (ici le méthacrylate de glycidyle) et en époxyde de formule (III) indiqués dans le tableau I ci-après.

## EXEMPLES 2 et 3

Le mode opératoire de l'exemple 1 est répété, à l'exception suivante près : le peroxyde d'hydrogène 20% aqueux est remplacé par du peroxyde d'hydrogène 8% aqueux. Les autres conditions opératoires et les résultats de la réaction sont rassemblés dans le tableau I.

EXEMPLE 4

Le mode opératoire de l'exemple 1 est répété, à l'exception suivante près : le peroxyde d'hydrogène 20% aqueux est remplacé par du peroxyde d'hydrogène 35% aqueux. Les autres conditions opératoires et les résultats de la réaction sont rassemblés dans le tableau I.

EXEMPLE 5

Le mode opératoire de l'exemple 1 est répété en remplaçant le méthacrylate d'allyle par l'acrylate d'allyle. Les conditions opératoires et les résultats de la réaction sont rassemblés dans le tableau I. L'époxyde de formule (II) formé ici est l'acrylate de glycidyle.

EXEMPLE 6

Le mode opératoire de l'exemple 1 est répété en remplaçant le méthacrylate d'allyle par le méthacrylate de crotyle (obtenu par transestérification de l'alcool crotylique sur le méthacrylate de méthyle et distillé avant emploi). Les conditions opératoires et les résultats de la réaction sont rassemblés dans le tableau I.

EXEMPLE 7

Le mode opératoire de l'exemple 1 est répété en remplaçant le méthacrylate d'allyle par le méthacrylate de décényle (obtenu par estérification du décène-ol-1 au moyen de chlorure de méthacryloyle). Les conditions opératoires et les résultats de la réaction sont rassemblés dans le tableau I.

EXEMPLE 8

Le mode opératoire de l'exemple 1 est répété en remplaçant le méthacrylate d'allyle par l'acrylate de décényle (obtenue par estérification du décène-ol-1 au moyen de chlorure d'acryloyle). Les conditions opératoires et les résultats de la réaction sont rassemblés dans le tableau I.

EXEMPLE 9

Le mode opératoire de l'exemple 1 est répété en remplaçant le méthacrylate d'allyle par le méthacrylate de dicyclopentadiényle (commercialisé par la demanderesse). Les conditions opératoires et les résultats de la réaction sont rassemblés dans le tableau I.

EXEMPLE 10

Le mode opératoire de l'exemple 1 est répété en remplaçant le méthacrylate d'allyle par l'acrylate de dicyclopentadiényle. Les conditions opératoires et les résultats de la réaction sont rassemblés dans le tableau I.

EXEMPLE 11

Le mode opératoire de l'exemple 1 est répété en remplaçant le méthacrylate d'allyle par le méthacrylate d'éthylidènenorbornène (commercialisé par la demanderesse). Les conditions opératoires et les résultats de la réaction sont rassemblés dans le tableau I.

EXEMPLE 12

Le mode opératoire de l'exemple 1 est répété en remplaçant le méthacrylate d'allyle par l'acrylate d'éthylidènenorbornène (commercialisé par la demanderesse). Les conditions opératoires et les résultats de la réaction sont rassemblés dans le tableau I.

EXEMPLE 13

Le mode opératoire de l'exemple 1 est répété en remplaçant le méthacrylate d'allyle par l'acrylate de dicyclopentadiényloxyéthanol (commercialisé par la demanderesse). Les conditions opératoires et les résultats

de la réaction sont rassemblés dans le tableau I.

TABLEAU I

| Exemple | pH | T | t | C | S(II) | S(III) |
|---|---|---|---|---|---|---|
| 1 | 1,9 | 26 | 255 | 90 | 67 | 2,8 |
| 2 | 2,5 | 20 | 96 | 53 | 58 | 6,4 |
| 3 | 3,5 | 20 | 160 | 33 | 74 | 10 |
| 4 | 1,7 | 27 | 160 | 72 | 64 | 4,2 |
| 5 | 2,5 | 25 | 327 | 100 | 70 | 3,5 |
| 6 | 1,7 | 28 | 48 | 87 | 95 | 1,7 |
| 7 | 1,7 | 28 | 29 | 74 | 69 | 2,2 |
| 8 | 1,7 | 28 | 44 | 92 | 94 | 1,4 |
| 9 | 1,7 | 28 | 6 | 95 | 100 | 0 |
| 10 | 1,7 | 30 | 6 | 96 | 100 | 0 |
| 11 | 1,7 | 28 | 5 | 99 | 100 | 0 |
| 12 | 1,7 | 28 | 4 | 99 | 100 | 0 |
| 13 | 1,7 | 25 | 18 | 95 | 100 | 0 |

EXEMPLE 14

Une solution constituée de 1 millimole de tungstate de sodium $Na_2WO_4$, $2H_2O$, de 1,7 millimole d'acide phosphorique 34% et de 40 millimoles de peroxyde d'hydrogène 20% aqueux, dont le pH est ajusté à 1,7 par une solution d'acide sulfurique 30%, est additionnée goutte à goutte, en 5 minutes, à une solution constituée de 0,4 millimole de chlorure de tricaprylylméthylammonium et de 20 millimoles de méthacrylate de cinnamyle (obtenu par transestérification de l'alcool cinnamique sur le méthacrylate de méthyle et distillé avant emploi) dans 10 millilitres de chlorure de méthylène. Le mélange est placé sous vive agitation à la température T (exprimée en degrés Celsius) indiquée au tableau II ci-après. Quelques minutes après l'addition, une coloration jaune apparaît puis s'atténue. La réaction est suivie en chromatographie en phase gazeuse par analyse de prélèvements réguliers. A l'issue du temps t (exprimé en heures) indiqué dans le tableau II ci-après, une solution acide de sulfate de fer est ajoutée au mélange réactionnel afin de détruire les peroxydes présents dans le milieu. A ce stade, sont calculés le taux de conversion de la réaction C (exprimé en %) ainsi que les sélectivités S en époxyde de formule (II) et en époxyde de formule (III) indiqués dans le tableau II ci-après.

EXEMPLE 15

Le mode opératoire de l'exemple 14 est répété en remplaçant le méthacrylate de cinnamyle par le méthacrylate d'allyle (obtenu par estérification de l'alcool allylique au moyen du chlorure de méthacrylate et distillé avant emploi). Les conditions opératoires et les résultats sont rassemblés dans le tableau II.

EXEMPLE 16

Le mode opératoire de l'exemple 14 est répété en remplaçant le méthacrylate de cinnamyle par le méthacrylate de dicyclopentadiényloxyéthanle (commercialisé par la demanderesse). Les conditions opératoires et

les résultats de la réaction sont rassemblés dans le tableau II.

EXEMPLE 17

Le mode opératoire de l'exemple 14 est répété en remplaçant le méthacrylate de cinnamyle par le méthacrylate de vinylnorbornyle (commercialisé par la demanderesse). Les conditions opératoires et les résultats de la réaction sont rassemblés dans le tableau II.

EXEMPLE 18

Le mode opératoire de l'exemple 14 est répété en remplaçant le méthacrylate de cinnamyle par l'acrylate de vinylnorbornyle. Les conditions opératoires et les résultats de la réaction sont rassemblés dans le tableau II.

EXEMPLE 19

Le mode opératoire de l'exemple 16 est répété en l'absence d'acide phosphorique 34%. Les conditions opératoires et les résultats de la réaction sont rassemblés dans le tableau II.

EXEMPLES 20 à 26

Le mode opératoire de l'exemple 16 est répété en faisant varier la nature de l'agent de transfert de phase qui est respectivement choisi comme suit :

- chlorure de tétrabutylammonium (exemple 20)
- bromure de tétrabutylammonium (exemple 21)
- iodure de tétrabutylammonium (exemple 22)
- hydrogénosulfate de tétrabutylammonium (exemple 23)
- bromure de tétrabutylphosphonium (exemple 24)
- chlorure de tétrabutylphosphonium (exemple 25)
- bromure de tributylhexadécylphosphonium (exemple 26)

Les conditions opératoires et les résultats de la réaction sont rassemblés dans le tableau II.

EXEMPLES 27 et 28

Le mode opératoire de l'exemple 16 est répété en faisant varier la concentration du peroxyde d'hydrogène aqueux de 8% (exemple 27) à 35% (exemple 28). Les conditions opératoires et les résultats de la réaction sont rassemblés dans le tableau II.

EXEMPLE 29

Le mode opératoire de l'exemple 16 est répété en remplaçant le pertungstate de sodium par la même quantité molaire de molybdate de sodium $Na_2MoO_4$. Les conditions opératoires et les résultats de la réaction sont rassemblés dans le tableau II.

TABLEAU II

| Exemple | T | t | C | S(II) | S(III) |
|---|---|---|---|---|---|
| 14 | 40 | 49 | 82 | 96 | 1,8 |
| 15 | 38 | 11 | 89 | 90 | 2,4 |
| 16 | 40 | 2,5 | 97 | 100 | 0 |
| 17 | 30 | 22 | 80 | 94 | 0 |
| 18 | 40 | 40 | 81 | 100 | 0 |
| 19 | 40 | 7 | 99,4 | 100 | 0 |
| 20 | 20 | 15 | 96 | 100 | 0 |
| 21 | 20 | 14 | 94 | 100 | 0 |
| 22 | 20 | 15 | 85,5 | 100 | 0 |
| 23 | 20 | 15 | 85,5 | 100 | 0 |
| 24 | 20 | 15 | 98,5 | 100 | 0 |
| 25 | 20 | 15 | 98 | 100 | 0 |
| 26 | 20 | 15 | 95,5 | 100 | 0 |
| 27 | 20 | 2,5 | 91 | 100 | 0 |
| 28 | 20 | 2 | 94 | 100 | 0 |
| 29 | 20 | 234 | 61 | 100 | 0 |

## Revendications

**1.** Procédé d'époxydation d'un composé (méth)acrylique insaturé de formule :

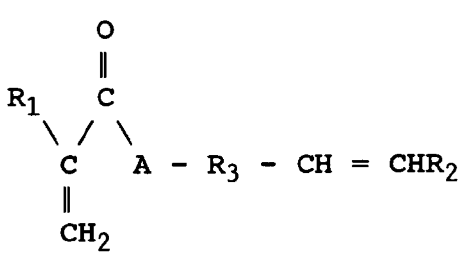

(I)

dans laquelle :

- $R_1$ est choisi parmi l'atome d'hydrogène et les radicaux alkyle ayant de 1 à 5 atomes de carbone ;
- $R_2$ est choisi parmi l'atome d'hydrogène, les radicaux alkyle en $C_1$-$C_{12}$ et les radicaux aryle en $C_6$-$C_{14}$ ;
- $R_3$ est un radical alkylène linéaire, ramifié ou cyclique, ayant de 1 à 12 atomes de carbone, ou un radical oxyalkylène de formule $-(CH_2)_m-O-R_4-$, avec $1 \leqq m \leqq 11$ et $R_4$ désignant un radical alkylène linéaire, ramifié ou cyclique, l'un des atomes de carbone de $R_2$, lorsque celui-ci est un radical alkyle, pouvant être lié à l'un des atomes de carbone de $R_3$ pour former un cycle ; et
- A est choisi parmi les atomes d'oxygène et de soufre et les radicaux NH et $NR_5$, $R_5$ étant un groupe alkyle ayant de 1 à 12 atomes de carbone,

par action de peroxyde d'hydrogène sur ledit composé (méth)acrylique insaturé, en présence d'au moins un catalyseur,
caractérisé par le fait qu'on conduit la réaction à une température comprise entre 10°C et 50°C, en présence d'au moins un molybdate alcalin et/ou tungstate alcalin comme catalyseur et en présence d'au moins un agent de transfert de phase, le rapport molaire entre le peroxyde d'hydrogène et le composé (méth)acrylique insaturé étant d'au moins une mole de peroxyde d'hydrogène pour une mole de composé (méth)acrylique insaturé, la concentration du peroxyde d'hydrogène étant comprise entre 5 et 50%, et le pH du milieu réactionnel étant compris entre 1 et 3,5, ladite réaction conduisant de façon sélective, totalement ou majoritairement, à l'époxyde de formule (II) :

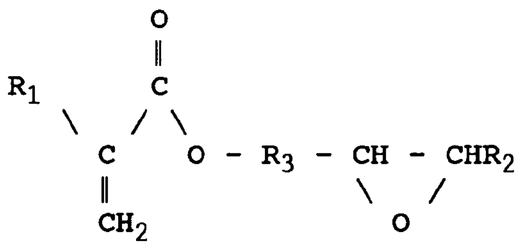
(II)

2. Procédé selon la revendication 1, caractérisé en ce que la réaction est effectuée en présence d'un solvant organique.

3. Procédé selon l'une des revendications 1 et 2, caractérisé en ce que l'agent de transfert de phase est choisi parmi les sels d'ammonium et de phosphonium quaternaires.

4. Procédé selon l'une des revendications 1 à 3, caractérisé en ce que le rapport molaire entre le peroxyde d'hydrogène et le composé insaturé est de 1,5 à 3 moles de peroxyde d'hydrogène pour une mole de composé (méth)acrylique insaturé.

5. Procédé selon l'une des revendications 1 à 4, caractérisé en ce que la concentration du peroxyde d'hydrogène est comprise entre 10% et 35%.

6. Procédé selon l'une des revendications 1 à 5, caractérisé en ce que le pH du milieu réactionnel est choisi entre 1,5 et 2,5.

7. Procédé selon l'une des revendications 1 à 6, caractérisé en ce que le catalyseur est utilisé à raison d'au moins 0,1% en moles par rapport au composé à époxyder.

8. Procédé selon l'une des revendications 1 à 7, caractérisé en ce que l'agent de transfert de phase est utilisé à raison d'au moins 0,5% en moles par rapport au composé à époxyder.

**Patentansprüche**

1. Verfahren zur Epoxidation einer ungesättigten (Meth)acrylverbindung der Formel

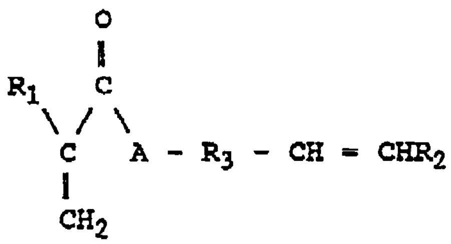
(I)

in der

- $R_1$ ausgewählt ist aus der Gruppe bestehend aus dem Wasserstoffatom und den Alkylresten mit 1 bis 5 Kohlenstoffatomen,

- $R_2$ ausgewählt ist aus der Gruppe bestehend aus dem Wasserstoffatom, den $C_1$-$C_{12}$-Alkylresten und den $C_6$-$C_{14}$-Arylresten,
- $R_3$ ein linearer, verzweigter oder cyklischer Alkylenrest mit 1 bis 12 Kohlenstoffatomen oder ein Oxyalkylenrest der Formel $-(CH_2)_m-O-R_4-$ ist, worin $1 \leqq m \leqq 11$ ist und $R_4$ einen linearen, verzweigten oder cyklischen Alkylenrest bezeichnet, wobei das eine Kohlenstoffatome von $R_2$, wenn letzteres ein Alkylrest ist, an das eine der Kohlenstoffatome von $R_3$ gebunden sein kann, um einen Ring zu bilden, und
- A ausgewählt ist aus der Gruppe bestehend aus den Sauerstoff- und Schwefelatomen und den Resten NH und $NR_5$, wobei $R_5$ eine Alkylgruppe mit 1 bis 12 Kohlenstoffatomen ist,

durch Einwirkung von Wasserstoffperoxid auf die genannte ungesättigte (Meth)acrylverbindung in Gegenwart mindestens eines Katalysators, dadurch gekennzeichnet, daß man die Reaktion bei einer Temperatur von 10°C bis 50°C in Gegenwart mindestens eines Alkalimolybdats und/oder Alkaliwolframats als Katalysator und in Gegenwart mindestens eines Phasenübertragungsmittels durchführt, wobei das Molverhältnis zwischen dem Wasserstoffperoxid und der ungesättigten (Meth)acrylverbindung mindestens ein Mol Wasserstoffperoxid zu einem Mol ungesättigte (Meth)acrylverbindung beträgt, die Wasserstoffperoxidkonzentration 5 bis 50% beträgt und der pH-Wert des Reaktionsmilieus 1 bis 3,5 beträgt, und wobei die Reaktion selektiv vollständig oder mehrheitlich zu dem Epoxid der Formel (II) führt:

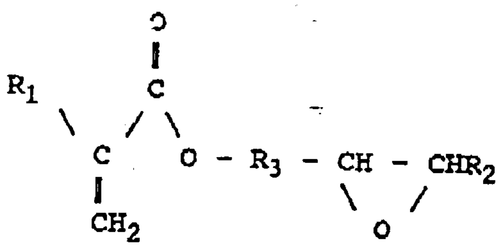

(II)

2. Verfahren nach Anspruch 1, dadurch gekennzeichnet, daß die Reaktion in Gegenwart eines organischen Lösungsmittels durchgeführt wird.

3. Verfahren nach Anspruch 1 oder 2, dadurch gekennzeichnet, daß das Phasenübertragungsmittel ausgewählt wird aus den quartären Ammonium- und Phosphoniumsalzen.

4. Verfahren nach einem der Ansprüche 1 bis 3, dadurch gekennzeichnet, daß das Molverhältnis zwischen dem Wasserstoffperoxid und der ungesättigten (Meth)acrylverbindung 1,5 bis 3 Mol Wasserstoffperoxid zu einem Mol ungesättigte (Meth)acrylverbindung beträgt.

5. Verfahren nach einem der Ansprüche 1 bis 4, dadurch gekennzeichnet, daß die Wasserstoffperoxidkonzentration 10 bis 35% beträgt.

6. Verfahren nach einem der Ansprüche 1 bis 5, dadurch gekennzeichnet, daß der pH-Wert des Reaktionsmilieus im Bereich von 1,5 bis 2,5 gewählt wird.

7. Verfahren nach einem der Ansprüche 1 bis 6, dadurch gekennzeichnet, daß der Katalysator in einer Menge von mindestens 0,1 Mol-%, bezogen auf die zu epoxidierende Verbindung, eingesetzt wird.

8. Verfahren nach einem der Ansprüche 1 bis 7, dadurch gekennzeichnet, daß das Phasenübertragungsmittel in einer Menge von mindestens 0,5 Mol-%, bezogen auf die zu epoxidierende Verbindung, eingesetzt wird.

## Claims

1. Process for the epoxidation of an unsaturated (meth)acrylic compound of formula:

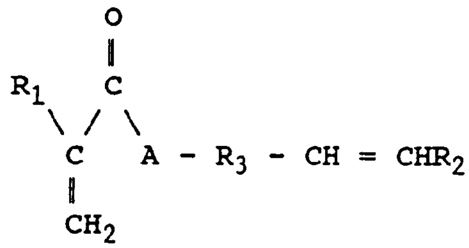

(I)

in which:

- $R_1$ is chosen from the hydrogen atom and the alkyl radicals having from 1 to 5 carbon atoms;
- $R_2$ is chosen from the hydrogen atom, the $C_1$-$C_{12}$ alkyl radicals and the $C_6$-$C_{14}$ aryl radicals;
- $R_3$ is a straight-chain, branched or cyclic alkylene radical having from 1 to 12 carbon atoms, or an oxyalkylene radical of formula $-(CH_2)_m-O-R_4-$, with $1 \leqq m \leqq 11$ and $R_4$ denoting a straight-chain, branched or cyclic alkylene radical, it being possible for one of the carbon atoms of $R_2$, when the latter is an alkyl radical, to be linked to one of the carbon atoms of $R_3$ to form a ring; and
- A is chosen from the oxygen and sulphur atoms and the radicals NH and $NR_5$, $R_5$ being an alkyl group having from 1 to 12 carbon atoms,

by the action of hydrogen peroxide on the said unsaturated (meth)acrylic compound, in the presence of at least one catalyst, characterised in that the reaction is carried out at a temperature of between 10°C and 50°C in the presence of at least one alkali metal molybdate and/or alkali metal tungstate, as catalyst, and in the presence of at least one phase transfer agent, the molar ratio between hydrogen peroxide and unsaturated (meth)acrylic compound being at least one mole of hydrogen peroxide per mole of unsaturated (meth)acrylic compound, the hydrogen peroxide concentration being between 5 and 50% and the pH of the reaction mixture being between 1 and 3.5, the said reaction leading selectively, completely or predominantly, to the epoxide of formula (II):

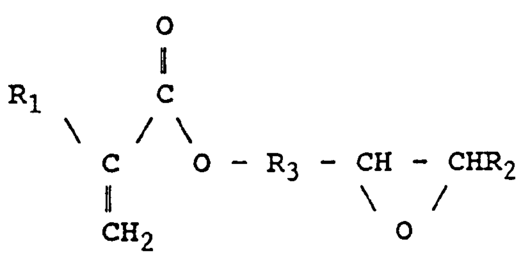

(II)

2. Process according to Claim 1, characterised in that the reaction is carried out in the presence of an organic solvent.

3. Process according to one of Claims 1 and 2, characterised in that the phase transfer agent is chosen from the quaternary ammonium and phosphonium salts.

4. Process according to one of Claims 1 to 3, characterised in that the molar ratio between hydrogen peroxide and the unsaturated compound is 1.5 to 3 mol of hydrogen peroxide per mole of unsaturated (meth)acrylic compound.

5. Process according to one of Claims 1 to 4, characterised in that the concentration of hydrogen peroxide is between 10% and 35%.

6. Process according to one of Claims 1 to 5, characterised in that the pH of the reaction mixture is chosen between 1.5 and 2.5.

7. Process according to one of Claims 1 to 6, characterised in that the catalyst is used in an amount of at least 0.1 mol % relative to the compound to be epoxidised.

8. Process according to one of Claims 1 to 7, characterised in that the phase transfer agent is used in an amount of at least 0.5 mol % relative to the compound to be epoxidised.